# EUROPEAN PATENT SPECIFICATION

(11) **EP 2 683 447 B1**
(45) Date of publication and mention of the grant of the patent: **09.11.2016**
(21) Application number: 12705875.8
(22) Date of filing: 27.02.2012
(51) Int. Cl.: A61K 8/02, A61K 8/19, A61Q 11/00, A61K 8/81, A61K 8/49

(54) **ORAL CARE COMPOSITIONS FOR TEMPORARY TOOTH WHITENING**
MUNDPFLEGEZUSAMMENSETZUNGEN FÜR DAS VORÜBERGEHENDE ZAHNBLEICHEN
COMPOSITIONS DE PROTECTION ORALE DE BLANCHIMENT TEMPORAIRE DES DENTS

(30) Priority: 11.03.2011 EP 11157896
(43) Date of publication of application: 15.01.2014
(73) Proprietor: Unilever PLC, London, Greater London EC4Y 0DY (GB); Unilever N.V., 3013 AL Rotterdam (NL)
(72) Inventor: GOLDING, Stephen, Wirral Merseyside CH63 3JW (GB); JARVIS, Adam, Peter, Wirral Merseyside CH63 3JW (GB); JOINER, Andrew, Wirral Merseyside CH63 3JW (GB); PHILPOTTS, Carole, Jane, Wirral Merseyside CH63 3JW (GB); WHITTAKER, Jane, Wirral Merseyside CH63 3JW (GB)
(74) Representative: Tansley, Sally Elizabeth
(86) International application number: PCT/EP2012/053248
(87) International publication number: WO 2012/123241

(56) References cited:
- EP-A1- 0 321 650
- EP-A1- 1 935 395
- WO-A2-2006/065529
- WO-A2-2006/065530

## Description

### Field of the Invention

The present invention is concerned with oral care compositions. More particularly, the present invention is concerned with oral care compositions containing a particulate tooth surface whitening agent.

### Background of the Invention

The colour of the teeth is influenced by a combination of their intrinsic colour and the presence of any extrinsic stains that may form on the tooth surface. Extrinsic colour is linked with the adsorption of materials into the acquired pellicle on the surface of enamel, which ultimately cause staining. Factors that influence extrinsic stain formation include poor tooth brushing technique, smoking, dietary intake of coloured foods (e.g. red wine), subject age and the use of certain cationic agents such as chlorhexidine or metal salts like tin and iron.

Consumers have always had a strong desire for white teeth and many individuals are dissatisfied with their current tooth colour. This desire for whiter teeth has given rise to a growing trend in the increased use of tooth whitening products.

Current whitening toothpastes rely on optimised abrasive and chemical components to maximise stain removal and prevention. During brushing, abrasive particles become temporarily trapped between the toothbrush and the stained tooth surface and abrade away the stain. Chemical components may also be used, usually in conjunction with abrasive particles, and include calcium chelators, polymers, surfactants, enzymes and oxidising agents.

EP 1 935 395 describes a novel optical approach to tooth whitening. On brushing with the toothpaste described in this publication, a blue pigment (in particular blue covarine) is deposited onto the tooth surface, where it is able to change the optical effects of the tooth surface, and enhance the measurement and perception of tooth whiteness. This toothpaste is intended to produce a temporary tooth whitening effect that can be reapplied as frequently as desired, as it contains no harsh chemicals, but is not intended to produce any permanent changes to the colour of the teeth. Deposition of the blue pigment onto the tooth surface is enhanced by the use of a deposition aid which is most preferably a GANTREZ® type polymer, i.e. a co-polymer of maleic anhydride with methyl vinyl ether.

The present inventors have now found that the temporary tooth whitening effects delivered by the system described in EP 1 935 395 can be enhanced by the use of certain poly-(sulphonic acid) polymers as deposition aids.

### Summary of the Invention

The present invention provides an oral care composition suitable for delivering a temporary whitening effect to the surface of teeth, the composition comprising:
a continuous phase comprising water or polyhydric alcohol or a mixture thereof;
a particulate tooth surface whitening agent which is dispersed in the continuous phase, and
a deposition aid for the particulate tooth surface whitening agent;
characterised in that the deposition aid is a poly-(sulphonic acid) polymer having:

   -[CH₂-C(R)(C(O)NH-C(R¹)(R²)-CH₂-SO₃X)]-

   monomers in its structure, where R is hydrogen or methyl; X is selected from hydrogen, ammonium, alkali metals or alkaline earth metals; and R¹ and R² are each independently selected from hydrogen and alkyl groups of 1 to 4 carbon atoms;
   and in which the particulate tooth surface whitening agent is a phthalocyanine blue pigment.

### Detailed Description of the Invention

### Tooth Surface Whitening Agent

The composition of the invention comprises a particulate tooth surface whitening agent which is dispersed in the continuous phase of the composition.

The particulate tooth surface whitening agent functions by being deposited onto the teeth and thereby changing their appearance. The deposition of the particulate tooth surface whitening agent onto the teeth is aided by the deposition aid.

The particulate tooth surface whitening agent is a phthalocyanine blue pigment.

Phthalocyanines are organometallic compounds containing four symmetrically arranged isoindole rings connected in a 16-membered ring linking with alternate carbon and nitrogen atoms. Most phthalocyanines contain a central, co-ordinated metal ion such as copper. Copper phthalocyanines have the basic structure:

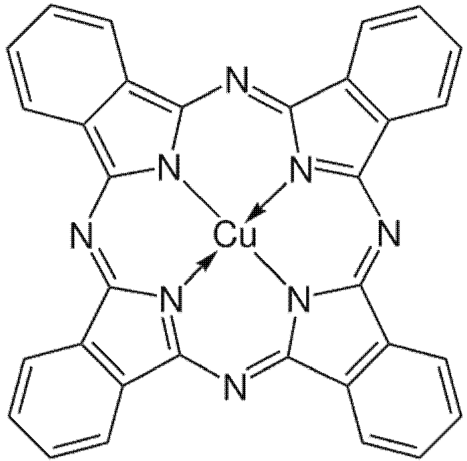

Phthalocyanine blue pigments exhibit more than one crystal modification, which differ in terms of coloristics. Methods have been developed to phase-stabilise the phthalocyanine pigment molecule in order to prevent conversion to a different crystal modification. An example is minor chemical modification of the molecule, for instance partial chlorination. Methods have also been developed to stabilise the phthalocyanine pigment molecule against flocculation during pigment application. An example is admixture of other agents to the molecule, such as surface active additives to the pigment molecule.

The following pigments are illustrative phthalocyanine blue pigments:

| C.I. Generic Name | C.I. Constitution Number | Crystal modification; Type | Number of halogen atoms |
|---|---|---|---|
| Pigment Blue 15 | 74 160 | alpha | - |
| Pigment Blue 15:1 | 74 160 | alpha; phase stabilised | 0.5-1 Cl |
| Pigment Blue 15:2 | 74 160 | alpha; phase & flocculation stabilised | 0.5-1 Cl |
| Pigment Blue 15:3 | 74 160 | beta | - |
| Pigment Blue 15:4 | 74 160 | beta; flocculation stabilised | - |
| Pigment Blue 15:6 | 74 160 | epsilon | - |
| Pigment Blue 16 | 74 100 | gamma; metal-free | - |

The most preferred particulate tooth surface whitening agents for use in the invention are those alpha copper phthalocyanines which are designated as C.I. Pigment Blue 15, C.I. Pigment Blue 15:1 or C.I. Pigment Blue 15:2 respectively, as described in the above Table. A commercially available example is Covarine Blue W 6795, ex Sensient Cosmetic Technologies/LCW.

Mixtures of any of the above described materials may also be used.

The amount of particulate tooth surface whitening agent (as defined above) in compositions of the invention suitably ranges from 0.001 to 3%, preferably from 0.001 to 1%, more preferably from 0.005 to 0.3% by total weight particulate tooth surface whitening agent (as defined above) based on the total weight of the composition.

### Deposition Aid

The composition of the invention comprises a deposition aid for the particulate tooth surface whitening agent. The deposition aid is a poly-(sulphonic acid) polymer having:

-[CH₂-C(R)(C(O)NH-C(R¹)(R²)-CH₂-SO₃X)]-

monomers in its structure, where R is hydrogen or methyl; X is selected from hydrogen, ammonium, alkali metals or alkaline earth metals; and R¹ and R² are each independently selected from hydrogen and alkyl groups of 1 to 4 carbon atoms.

Suitable poly-(sulphonic acid) polymers for use as deposition aids in the invention generally have a weight average molecular weight ranging from 50,000 to 10,000,000, preferably from 80,000 to 8,000,000, and more preferably from 100,000 to 7,000,000. A suitable method to determine the weight average molecular weight of such polymeric materials is gel permeation chromatography against a polyethylene glycol standard.

Suitable poly-(sulphonic acid) polymers for use as deposition aids in the invention may be water-soluble or water-dispersible. The term "water-soluble or water-dispersible" in the context of this invention generally means polymers which, when introduced into an aqueous phase at 25° C, at a concentration by mass equal to 1%, allow the obtaining of a macroscopically homogeneous and transparent solution, that is to say having a maximum light transmittance value, at a wavelength equal to 500 nm, through a sample 1 cm thick, of at least 60%, preferably of at least 70%.

The poly-(sulphonic acid) polymers for use as deposition aids in the invention may also be amphiphilic, i.e. they contain both a hydrophilic part or moiety and a hydrophobic part or moiety containing at least one fatty chain.

The term "deposition aid" in the context of this invention generally means a material which aids deposition of the particulate tooth surface whitening agent from the continuous phase of the composition onto the surface of teeth during use of the composition. Use of the composition in the context of this invention typically involves application of the composition to the oral cavity, followed by brushing and/or rinsing).

Suitable deposition aids work by having affinity for both the particulate tooth surface whitening agent (as defined above) and the surface of the teeth.

Preferred deposition aids are able to aid the deposition of the particulate tooth surface whitening agent onto the teeth such that tooth surface whiteness is enhanced by at least 20% and more preferably by at least 100%, in comparison to the value obtained for teeth treated in an equivalent manner with a control formulation using the same amount of particulate tooth surface whitening agent in the absence of the deposition aid.

A convenient measure of enhanced tooth surface whiteness is delta b* measured using a chromameter. A negative value of delta b* indicates a yellow to blue colour shift which has been shown to be one of the primary drivers of tooth surface whiteness as perceived by the consumer.

Accordingly, preferred deposition aids are able to aid the deposition of the particulate tooth surface whitening agent onto the teeth such that the negative value of delta b* is augmented by at least 20% and more preferably by at least 100%, in comparison to the value obtained for teeth treated in an equivalent manner with a control formulation using the same amount of particulate tooth surface whitening agent in the absence of the deposition aid.

A simple empirical model for establishing the effect of the deposition aid is as follows:
Polished hydroxyapatite discs are first placed in sterile human saliva for 2 hours to allow a pellicle to form. The discs are then rinsed in water and baseline colour measurements made (using, for example, a Minolta chromameter CR300). The discs are then brushed with either (a) a suspension of the particulate tooth surface whitening agent in water; or (b) a suspension of the particulate tooth surface whitening agent in water at the same concentration as in (a), together with the deposition aid. The brushing is best performed using a brushing machine. Following rinsing, the colour of the discs is then re-measured and the change in delta b* is recorded for both treatment (a) and treatment (b). From a comparison of these data, the effect of the deposition aid is readily seen.

A preferred class of poly-(sulphonic acid) polymer for use as a deposition aid in the invention has:

-[CH₂-C(R)(C(O)NH-C(R¹)(R²)-CH₂-SO₃X)]-

monomers in its structure, in which R is hydrogen, R¹ and R² are each independently selected from alkyl groups of 1 to 3 carbon atoms and X represents hydrogen, ammonium or an alkali metal.

A particularly preferred class of poly-(sulphonic acid) polymers for use as deposition aids in the invention has:

-[CH₂-C(R)(C(O)NH-C(R¹)(R²)-CH₂-SO₃X)]-

monomers in its structure, in which R is hydrogen, R¹ and R² are each methyl and X represents hydrogen, ammonium, sodium or potassium. Such monomers are hereinafter termed 2-acrylamido-2-methylpropane sulphonic acid or AMPS® monomers.

Suitable poly-(sulphonic acid) polymers of the above class may be homopolymers or copolymers of 2-acrylamido-2-methylpropane sulphonic acid (AMPS®) monomer. The polymers may be crosslinked or non-crosslinked. When the polymers are crosslinked, the crosslinking agents may be selected from among the olefinically polyunsaturated compounds commonly used for the crosslinking of polymers obtained by free-radical polymerization. Examples of cross-linking agents include methylenebisacrylamide, allyl methacrylate and trimethylolpropane triacrylate (TMPTA).

Examples of AMPS® copolymers include copolymers of AMPS® with one or more anionic comonomers and/or one or more nonionic comonomers.

Examples of suitable anionic comonomers include acrylic acid, methacrylic acid, beta-carboxyethyl acrylate, acryloxypropionic acid, itaconic acid, monoalkyl esters of itaconic acid, maleic acid, monoalkyl esters of maleic acid, crotonic acid, fumaric acid, monoalkyl esters of fumaric acid, and their salts.

Preferred examples of anionic comonomers include acrylic acid and itaconic acid and their salts.

Examples of suitable nonionic comonomers include acrylamide, methacrylamide, N-vinylacetamide and N-methyl-N-vinylacetamide, N-vinylformamide and N-methyl-N-vinyl formamide, N-alkyl and N,N-dialkyl acrylamides, N-alkyl and N,N-dialkyl methacrylamides, alkyl acrylates, alkyl methacrylates, benzyl acrylate, benzyl methacrylate, N-vinyllactams containing a cyclic alkyl group having from 4 to 9 carbon atoms (such as N-vinyl pyrrolidone, N-vinyl butyrolactam and N-vinyl caprolactam), vinyl esters, vinyl alcohols, propylene glycols or ethylene glycols, in which the alkyl groups of these monomers preferably contain 1 to 7, more preferably 1 to 3 carbon atoms. Other suitable nonionic comonomers include esters of acrylic acid or methacrylic acid with alkyl ethoxylates, such as monomers of the following formula:

-[CH₂-C(R¹)(C(O)-O-[CH₂-CH₂-O]ₙ-R²)]-

in which n is an integer ranging from 3 to 100, preferably from 3 to 50, more preferably from 7 to 25, R¹ is hydrogen or a methyl radical and R² denotes a linear or branched alkyl radical containing from 6 to 30 carbon atoms, preferably from 10 to 22 carbon atoms, more preferably from 14 to 22 carbon atoms.

Preferred examples of nonionic comonomers include acrylamide, methacrylamide, N-vinylformamide, N-methyl and N,N-dimethyl acrylamides, N-methyl and N,N-dimethyl methacrylamides, N-ethyl and N,N-diethyl acrylamides, N-ethyl and N,N-diethyl methacrylamides, N-vinyl pyrrolidone and esters of acrylic or methacrylic acid with polyethoxylated C₁₄-C₂₂ fatty alcohols containing from 7 to 25 ethoxy groups.

Suitable AMPS® copolymers for use in the invention are commercially available, for example from Clariant under the ARISTOFLEX® tradename, such as ARISTOFLEX® AVC (INCI name: ammonium acryloyldimethyltaurate/VP Copolymer), ARISTOFLEX® AVS (INCI name: sodium acryloyldimethyltaurate/VP Copolymer), ARISTOFLEX® HMB (INCI name: ammonium acryloyldimethyltaurate/Beheneth-25 Methacrylate Crosspolymer) and ARISTOFLEX® TAC (INCI name: ammonium acryloyldimethyltaurate/Carboxyethyl Acrylate Crosspolymer).

Preferred 2-acrylamido-2-methylpropane sulphonic acid (AMPS®) copolymers for use in the invention comprise at least 25%, more preferably at least 50% and most preferably at least 75% by total weight AMPS® monomer based on the total weight of the polymer.

Particularly good results have been obtained with AMPS® homopolymer.

Mixtures of any of the above described materials may also be used.

The amount of deposition aid (as defined above) in compositions of the invention suitably ranges from 0.001 to 5.0%, preferably from 0.005 to 3.0%, more preferably from 0.01 to 1.0% by total weight deposition aid (as defined above) based on the total weight of the composition.

### Product Form

The composition of the invention comprises a continuous phase comprising water or polyhydric alcohol or a mixture thereof.

Examples of suitable product forms for compositions of the invention include dentifrices, mouthwashes, chewing gums, lozenges, bioadhesive patches or strips, mouthsprays and liquid formulations, such as paints or lacquers, which are designed to be applied directly to the tooth surface using an applicator such as a brush or sponge tip applicator.

Preferred product forms for compositions of the invention are those which are suitable for brushing and/or rinsing the surfaces of the oral cavity.

In such preferred product forms, the amount of water and/or polyhydric alcohol will generally be at least 10%, preferably at least 30%, more preferably at least 50% by total weight water and/or polyhydric alcohol based on the total weight of the composition.

An example of a preferred type of product form in the context of the present invention is a dentifrice. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

A dentifrice composition according to the invention will usually contain a liquid continuous phase in an amount of from 40 to 99% by weight based on the total weight of the dentifrice. Such a liquid continuous phase will typically comprise a mixture of water and polyhydric alcohol in various relative amounts, with the amount of water generally ranging from 10 to 45% by weight (based on the total weight of the dentifrice) and the amount of polyhydric alcohol generally ranging from 30 to 70% by weight (based on the total weight of the dentifrice). Typical polyhydric alcohols include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof.

A dentifrice composition according to the invention will generally contain further ingredients to enhance performance and/or consumer acceptability such as abrasive cleaning agent, binder or thickening agent, and surfactant.

For example, a dentifrice will usually comprise an abrasive cleaning agent in an amount of from 3 to 75% by weight based on the total weight of the dentifrice. Suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof.

Furthermore, the dentifrice will usually contain a binder or thickening agent in an amount of from 0.5 to 10% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth), finely divided silicas, hectorites, colloidal magnesium aluminium silicates and mixtures thereof.

Furthermore, the dentifrice will usually contain a surfactant in an amount of from 0.2 to 5% by weight based on the total weight of the dentifrice. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

Another example of a preferred type of product form in the context of the present invention is a mouthwash. The term "mouthwash" generally denotes liquid formulations which are used to rinse the surfaces of the oral cavity and provide the user with a sensation of oral cleanliness and refreshment. The mouthwash is an oral composition that is not intentionally swallowed for purposes of systemic administration of therapeutic agents, but is applied to the oral cavity, used to treat the oral cavity and then expectorated.

A mouthwash composition according to the invention will usually contain an aqueous continuous phase. The amount of water generally ranges from 70 to 99% by weight based on the total weight of the mouthwash.

A mouthwash composition according to the invention will generally contain further ingredients to enhance performance and/or consumer acceptability, such as the humectants and surfactants mentioned above for dentifrices. The amount of humectant generally ranges from 5 to 20% by weight based on the total weight of the mouthwash and the amount of surfactant generally ranges from 0.1 to 5% by weight based on the total weight of the mouthwash.

Compositions of the present invention (such as in particular dentifrices or mouthwashes) may also contain further optional ingredients customary in the art such as fluoride ion sources, anticalculus agents, buffers, flavouring agents, sweetening agents, colouring agents, opacifying agents, preservatives, antisensitivity agents and antimicrobial agents.

The invention is further illustrated with reference to the following, non-limiting Examples.

### EXAMPLES

An evaluation was conducted in order to assess the delivery of blue pigment to a model tooth surface using poly-(sulphonic acid) polymers as deposition aids. The delivery was compared against a formulation which is representative of the preferred blue pigment & deposition aid combination which is described in EP 1 935 395 (i.e. 0.025% w/w Blue Covarine pigment plus 0.1 % w/w GANTREZ®S97 deposition aid ex ISP).

Accordingly, test formulations were prepared having the ingredients shown in Table 1 below.

All ingredients are expressed by weight percent of the total formulation, and as level of active ingredient.

**Table 1**

| Ingredient | Comparative Example | Example 1 | Example 2 |
|---|---|---|---|
| GANTREZ® S-97 | 0.1 | 0 | 0 |
| Poly(2-acrylamido-2-methyl-1-propanesulfonic acid)⁽¹⁾ | 0 | 0.1 | 0 |
| RHEOCARE®HSP-1180⁽²⁾ | 0 | 0 | 0.1 |
| Blue Covarine (C.I.74160); 40% a.i. | 0.025 | 0.025 | 0.025 |
| Water | to 100 | to 100 | to 100 |

| | | | |
|---|---|---|---|
| ⁽¹⁾ Average M_{w} 2,000,000, 15 wt% in H₂O, ex Sigma-Aldrich® ⁽²⁾ Poly(2-acrylamido-2-methyl-1-propanesulfonic acid), 14-18 wt% in H₂O, ex Cognis Care Chemicals | | | |

### Experimental Method

Hydroxyapatite (HA) discs were polished using P1200 grit
silicon carbide paper and thoroughly rinsed with water. The discs were immersed in sterilised whole human saliva for a minimum of two hours to allow a pellicle to form. The HA discs were removed from the saliva, rinsed with water and the baseline colour of the discs were measured with a colorimeter in the CIELAB mode. The HA discs were then placed in a brushing machine and 10ml of the test formulation added per disc. The discs were brushed for 1 minute under a brushing load of 375 g. After brushing the HA discs were rinsed in water and the colour of the discs re-measured. Changes in the colour co-ordinates b* and whiteness index (WIO) were calculated. The WIO index is an index which has been optimised specifically for the evaluation of whiteness in teeth (as described in Journal of Dentistry, Volume 36, Supplement 1, 2008, pages 2 to 7).

### Results

The changes in b* and whiteness (WIO) are shown in Table 2 below.

**Table 2**

| Formulation | Change in b* | Change in WIO |
|---|---|---|
| Comparative Example | -12.17 | 19.3 |
| Example 1 | -18.78 | 30.1 |
| Example 2 | -19.07 | 28.7 |

The results demonstrate the superior whitening effect which is delivered from the formulations of Examples 1 and 2, compared with the Comparative Example.

## Claims

1. An oral care composition suitable for delivering a temporary whitening effect to the surface of teeth, the composition comprising:
a continuous phase comprising water or polyhydric alcohol or a mixture thereof;
a particulate tooth surface whitening agent which is dispersed in the continuous phase, and
a deposition aid for the particulate tooth surface whitening agent;
**characterised in that** the deposition aid is a poly-(sulphonic acid) polymer having:
-[CH₂-C(R)(C(O)NH-C(R¹)(R²)-CH₂-SO₃X)]-
monomers in its structure, where R is hydrogen or methyl; X is selected from hydrogen, ammonium, alkali metals or alkaline earth metals; and R¹ and R² are each independently selected from hydrogen and alkyl groups of 1 to 4 carbon atoms;
and in which the particulate tooth surface whitening agent is a phthalocyanine blue pigment.

2. An oral care composition according to claim 1, in which the phthalocyanine blue pigment is selected from those alpha copper phthalocyanines which are designated as C.I.Pigment Blue 15, C.I. Pigment Blue 15:1 or C.I. Pigment Blue 15:2.

3. An oral care composition according to any preceding claim, in which the poly-(sulphonic acid) polymer
has:
-[CH₂-C(R)(C(O)NH-C(R¹)(R²)-CH₂-SO₃X)]-
monomers in its structure, in which R is hydrogen, R¹ and R² are each independently selected from alkyl groups of 1 to 3 carbon atoms and X represents hydrogen, ammonium or an alkali metal.

4. An oral care composition according to claim 3, in which the poly-(sulphonic acid) polymer has
-[CH₂-C(R)(C(O)NH-C(R¹)(R²)-CH₂-SO₃X)]-
monomers in its structure, in which R is hydrogen, R¹ and R² are each methyl and X represents hydrogen, ammonium, sodium or potassium.

5. An oral care composition according to claim 4, in which the poly-(sulphonic acid) polymer is a homopolymer of:
-[CH₂-C(R)(C(O)NH-C(R¹)(R²)-CH₂-SO₃X)]-
monomers, in which R is hydrogen, R¹ and R² are each methyl and X represents hydrogen, ammonium, sodium or potassium.

6. An oral care composition according to any preceding claim, in which the poly-(sulphonic acid) polymer has a weight average molecular weight ranging from 100,000 to 7,000,000.

7. An oral care composition according to any preceding claim, which is in the form of a dentifrice or a mouthwash.

## Patentansprüche

1. Mundpflegezusammensetzung, die geeignet ist, einen temporären Aufhelleffekt auf der Oberfläche von Zähnen zu liefern, wobei die Zusammensetzung umfasst:
eine kontinuierliche Phase, die Wasser oder mehrwertigen Alkohol oder eine Mischung davon umfasst,
ein teilchenförmiges Aufhellmittel für die Zahnoberfläche, das in der kontinuierlichen Phase dispergiert ist, und
ein Abscheidungshilfsmittel für das teilchenförmige Aufhellmittel für die Zahnoberfläche,
**dadurch gekennzeichnet, dass** das Abscheidungshilfsmittel ein Poly-(sulfonsäure)polymer mit
-[CH₂-C(R)(C(O)NH-C(R¹)(R²)-CH₂-SO₃X)]-
Monomeren in seiner Struktur ist,
worin R Wasserstoff oder Methyl ist, X aus Wasserstoff, Ammonium, Alkalimetallen oder Erdalkalimetallen ausgewählt ist und R¹ und R², jeweils unabhängig voneinander, unter Wasserstoff und Alkylgruppen mit 1 bis 4 Kohlenstoffatomen ausgewählt sind,
und in welcher das teilchenförmige Aufhellmittel für die Zahnoberfläche ein Phthalocyanin-Blaupigment ist.

2. Mundpflegezusammensetzung nach Anspruch 1, worin das Phthalocyanin-Blaupigment unter solchen alpha-Kupferphthalocyaninen ausgewählt ist, die als C. I. Pigment Blue 15, C. I. Pigment Blue 15:1 oder C. I. Pigment Blue 15:2 bezeichnet werden.

3. Mundpflegezusammensetzung nach irgendeinem vorhergehenden Anspruch, worin das Poly(sulfonsäure)polymer
-[CH₂-C(R)(C(O)NH-C(R¹)(R²)-CH₂-SO₃X)]-
Monomere in seiner Struktur aufweist, worin R Wasserstoff ist, R¹ und R², jeweils unabhängig voneinander, aus Alkylgruppen mit 1 bis 3 Kohlenstoffatomen ausgewählt sind und X Wasserstoff, Ammonium oder ein Alkalimetall darstellt.

4. Mundpflegezusammensetzung nach Anspruch 3, worin das Poly-(sulfonsäure)polymer
-[CH₂-C(R)(C(O)NH-C(R¹)(R²)-CH₂-SO₃X)]-
Monomere in seiner Struktur aufweist, worin R Wasserstoff ist, R1 und R2 jeweils Methyl sind und X Wasserstoff, Ammonium, Natrium oder Kalium darstellt.

5. Mundpflegezusammensetzung nach Anspruch 4, in welcher das Poly-(sulfonsäure)polymer ein Homopolymer von
-[CH₂-C(R)(C(O)NH-C(R¹)(R²)-CH₂-SO₃X)]-
Monomeren ist, in welchen R Wasserstoff ist, R¹ und R² jeweils Methyl sind und X Wasserstoff, Ammonium, Natrium oder Kalium darstellt.

6. Mundpflegezusammensetzung nach irgendeinem vorhergehenden Anspruch, worin das Poly(sulfonsäure)polymer ein gewichtsgemitteltes Molekulargewicht zwischen 100.000 und 7.000.000 aufweist.

7. Mundpflegezusammensetzung nach irgendeinem vorhergehenden Anspruch, welches in Form eines Zahnputzmittels oder eines Mundwassers vorliegt.

## Revendications

1. Composition pour soins bucco-dentaires capable d'exercer un effet blanchissant temporaire sur la surface des dents, la composition comprenant :
une phase continue comprenant de l'eau ou un alcool polyhydrique ou un mélange des deux ;
un agent de blanchiment de la surface des dents sous forme particulaire qui est dispersé dans la phase continue ; et
un auxiliaire de dépôt pour l'agent de blanchiment de la surface des dents sous forme particulaire ;
**caractérisée en ce que** l'auxiliaire de dépôt est un polymère d'acide poly(sulfonique) comportant des monomères
**-[CH₂-C(R)(C(O)NH-C(R¹)(R²)-CH₂-SO₃X)]-**
dans sa structure, où R est un atome d'hydrogène ou un méthyle ; X est choisi parmi un atome d'hydrogène, un ion ammonium, des métaux alcalins ou des métaux alcalino-terreux ; et R¹ et R² sont chacun indépendamment choisis parmi un atome d'hydrogène et des groupes alkyle ayant de 1 à 4 atomes de carbone ;
et dans laquelle l'agent de blanchiment de la surface des dents sous forme particulaire est un pigment bleu à la phtalocyanine.

2. Composition pour soins bucco-dentaires selon la revendication 1, dans laquelle le pigment bleu à la phtalocyanine est choisi parmi les alpha-phtalocyanines de cuivre qui sont appelées Pigment C.I. Blue 15, Pigment C.I. Blue 15.1 ou Pigment C.I. Blue 15.2.

3. Composition pour soins bucco-dentaires selon l'une quelconque des revendications précédentes, dans laquelle le polymère d'acide poly(sulfonique) comporte des monomères
**-[CH₂-C(R)(C(O)NH-C(R¹)(R²)-CH₂-SO₃X)]-**
dans sa structure, où R est un atome d'hydrogène, R¹ et R² sont chacun indépendamment choisis parmi des groupes alkyle ayant de 1 à 3 atomes de carbone et X représente un atome d'hydrogène, un ion ammonium ou un métal alcalin.

4. Composition pour soins bucco-dentaires selon la revendication 3, dans laquelle le polymère d'acide poly(sulfonique) comporte des monomères
**-[CH₂-C(R)(C(O)NH-C(R¹)(R²)-CH₂-SO₃X)]-**
dans sa structure, où R est un atome d'hydrogène, R¹ et R² sont chacun un méthyle et X représente un atome d'hydrogène, un ion ammonium, sodium ou potassium.

5. Composition pour soins bucco-dentaires selon la revendication 4, dans laquelle le polymère d'acide poly(sulfonique) est un homopolymère constitué de monomères
**-[CH₂-C(R)(C(O)NH-C(R¹)(R²)-CH₂-SO₃X)]-**
où R est un atome d'hydrogène, R¹ et R² sont chacun un méthyle et X représente un atome d'hydrogène, un ion ammonium, sodium ou potassium.

6. Composition pour soins bucco-dentaires selon l'une quelconque des revendications précédentes, dans laquelle le polymère d'acide poly(sulfonique) a un poids moléculaire moyen en poids de 100 000 à 7 000 000.

7. Composition pour soins bucco-dentaires selon l'une quelconque des revendications précédentes, qui est sous la forme d'un dentifrice ou d'un bain de bouche.
